# EUROPEAN PATENT SPECIFICATION

(11) **EP 4 335 405 B1**
(45) Date of publication and mention of the grant of the patent: **25.11.2026**
(21) Application number: 23217096.9
(22) Date of filing: 03.10.2019
(51) Int. Cl.: A61B 50/13, A61G 12/00, A61G 13/10, A61B 90/57, B62B 3/04, B62B 7/04

(54) **MOBILE SUPPORT AND STORAGE SYSTEM FOR A MEDICAL DEVICE**
MOBILES STÜTZ- UND AUFBEWAHRUNGSSYSTEM FÜR EINE MEDIZINISCHE VORRICHTUNG
SUPPORT MOBILE ET SYSTÈME DE STOCKAGE POUR UN DISPOSITIF MÉDICAL

(30) Priority: 05.10.2018 US 201816152811
(43) Date of publication of application: 13.03.2024
(62) Document number(s) of the earlier application(s) in accordance with Art. 76 EPC: 22194515.7 / 4 119 086; 19868438.3 / 3 843 654
(73) Proprietor: Siemens Healthineers Endovascular Robotics, Inc., Newton, MA 02466 (US)
(72) Inventor: KLEM, Eric, Lexington, MA, 02420 (US); ROW, Gordon, Waltham, MA, 02452 (US); LAING, Genevieve R. K., Medford, MA, 02215 (US); EVANS, Christopher O., Amherst, NH, 03031 (US); PARKER, Anthony Clegg, New Ipswich, NH, 03071 (US)
(74) Representative: HKW Intellectual Property PartG mbB

(56) References cited:
- EP-A1- 1 574 197
- EP-A1- 2 145 586
- WO-A1-00/09061
- US-A1- 2006 163 829
- US-B1- 10 034 721
- US-B1- 7 845 601

## Description

### CROSS-REFERENCE TO RELATED APPLICATIONS

This application claims priority to U.S. Application Serial No. 16/152,811.

### FIELD OF THE INVENTION

The present invention relates generally to the field of medical devices and more particularly to a mobile support and storage system for a medical device.

### BACKGROUND OF THE INVENTION

Medical beds or tables used in hospitals, clinics, doctor offices or other medical environments may include a rail operatively secured to the bed for supporting various medical devices. There may be circumstances where it is desirable to remove the medical device from the table between procedures. However, such devices may be heavy and manually installing and removing the medical devices can be difficult.

It would be desirable to provide a mobile support and storage system that is configured to facilitate installation and removal of a medical device with respect to a rail on a medical bed or table and that is also configured to transport and store the medical device.

US 7 845 601 B1 relates to a medical equipment transport system for supporting medical equipment while a patient is transported. The system comprises an equipment rack, such as an IV stand. While in the hospital room, the rack is mounted on a bracket supported on a mobile tower. When the patient is moved, the rack is transferred to a similar bracket on the bed. Transfer of the rack between bed and tower brackets is accomplished using a lift mechanism in the tower. The lift mechanism includes a bogie stabilized by radially-arranged bearings, enabling the tower to accept omni directional loading and moment forces. The rack is supported on each of the brackets by two vertically-aligned conical pins nested in tapered bushings, providing additional stability. A self-locking, self-releasing latch assembly prevents inadvertent dislocation of the rack. A customized adapter enables attachment of the bracket to any model bed without structurally altering it, simplifying installation and preserving warranties.

EP 2 145 586 A1 relates to an adapter for fixing a medical device to one or two support structures, wherein the adapter is constructed in three parts from a carrying part and two support parts, wherein the carrying part can be connected to the medical device, the first support part can be connected to a first support structure and the second support part can be connected to a second support structure, and the adapter can assume at least three states, wherein in the first state the carrying part is connected without play only to the first support part, in the second state the carrying part is connected without play only to the second support part and in the third state the carrying part is connected without play only to the first support part and in the third state the carrying part is connected without play only to the second support part. The support part is connected without play only to the first support part, in the second state the support part is connected without play only to the second support part and in the third state the support part is connected without play to the first support part and the second support part.

US 10 034 721 B1 relates to apparatuses and methods for transporting and coupling a robotic arm to a surgical table. In some embodiments, an arm cart may include a damping mechanism (e.g., a spring, a dashpot) configured to damp an impact force imparted to the robotic arm due to the arm contacting another object, such as a surgical table. In other embodiments, an arm cart may include a backstop with an inclined surface configured to damp an impact force imparted to the robotic arm. In other embodiments, the arm cart may include a compliant arm support that is bendable to damp an impact force imparted to a robotic arm. In some embodiments, the arm cart may include a damping mechanism configured to move from an extended position to a retracted position to permit a robotic arm to couple to a surgical table.

EP 1 574 197 A1 relates to a coupling mechanism of a mobile equipment cart for coupling an equipment carrier to a stationary receiving unit of a medical ceiling stand or to a mobile equipment cart, wherein the equipment carrier has at least one suspension device, the stationary receiving unit has at least one stationary receiving element for receiving the suspension device, and the mobile equipment trolley has a column and a receiving element which is movable relative thereto and has at least one receiving element for receiving the suspension device, the coupling mechanism comprising a movement device for moving the movable receiving element.

WO 00/09061 A1 relates to a portable patient care device transfer system that includes an equipment support element that can be rapidly and safely transferred between a mobile base element and the patient's bed. In one embodiment, the base element includes a rack coupling mechanism movably secured to a vertical structural member. The equipment support element includes a rack supported by a support member extending from a housing, which includes a bed coupling mechanism and a base coupling mechanism located on the housing bottom. The bed coupling mechanism is engageable to the bed and the base coupling mechanism is engageable to the rack coupling mechanism of the base element. To transfer the equipment support element from the bed to the base element, the rack coupling mechanism on the base element is lowered and aligned with the base coupling mechanism of the housing. The rack coupling mechanism is then raised to lift the equipment support element until it is disengaged from the bed and supported by the base element, which can then be moved away from the bed. The process is reversed to transfer the equipment support element back to the bed.

US 2006/163829 A1 relates to a patient support platform that provides a solution for healthcare facilities and nursing staff to address patient and staff safety, patient mobility, patient comfort, the availability of patient information, monitoring drugs and therapy provided, and controlling health care expenses. The patient support platform preferably includes a transmission system that allows the patient and/or medical staff member to choose a stop, walk or roll mode. The transmission system preferably includes a drag wheel for applying a braking force in response to a voltage generated by a braking motor. The platform supports a plurality of devices that may be attached or associated with a patient throughout their stay at a healthcare facility. The support platform also preferably includes a mechanism for releasably attaching the support platform to another structure, such as a bed. Embodiments of the present invention include multiple non-medical uses of the platform.

### SUMMARY OF THE INVENTION

The invention is defined in the appended set of claims. Aspects and embodiments are described in the following to illustrate the invention. In accordance with an aspect, a mobile support system for a medical device having an arm with a base, includes a body comprising a housing having a top surface, a first end and a second end and a carriage positioned within the housing, a mechanism coupled to the carriage and configured to cause movement of the carriage, a set of wheels coupled to the housing, a support arm coupled to the carriage and extending vertically upward from the top surface of the housing, the support arm configured to support the arm of the medical device, a mounting block coupled to the carriage proximate to the support arm, the mounting block configured to couple with the base of the arm of the medical device, a first rail detect guide located on the top surface at the second end of the housing and a second rail detect guide located on the top surface at the second end of the housing, wherein the first rail detect guide and the second rail detect guide are configured to unlock the mechanism used to cause movement of the carriage when contact is made between the first rail detect guide and the second rail detect guide and a surface.

In accordance with another aspect, a mobile support system for a medical device having an arm with a base, includes a body comprising a housing having a top surface and a carriage positioned within the housing, a mechanism coupled to the carriage and configured to cause movement of the carriage, a set of wheels coupled to the housing, a floor brake coupled to the housing, the floor brake configured to be actuated in response to movement of the carriage, a support arm coupled to the carriage and extending vertically upward from the top surface of the housing, the support arm configured to support the arm of the medical device and a mounting block coupled to the carriage proximate to the support arm, the mounting block configured to couple with the base of the arm of the medical device.

In accordance with another aspect, a mobile support system includes a medical device having an arm with a base, the base comprising a rail detect lever configured to be actuated when contact is made between the rail detect lever and a surface, a body comprising a housing and a carriage positioned within the housing, a support arm coupled to the carriage and extending vertically upward from the housing, the support arm configured to support the arm of the medical device and a mounting block coupled to the carriage proximate to the support arm, the mounting block configured to couple with the base of the arm of the medical device.

In accordance with another aspect, a mobile support system includes a medical device having an arm with a base, the base comprising a brake and a brake release coupled to the brake, a body comprising a housing and a carriage positioned within the housing, a support arm coupled to the carriage and extending vertically upward from the housing, the support arm configured to support the arm of the medical device and a mounting block coupled to the carriage proximate to the support arm, the mounting block configured to couple with the base of the arm of the medical device, the mounting block configured to actuate the brake release when the mounting block engages the base of the arm.

In accordance with an embodiment, a mobile support system includes a medical device having an arm with a base, the base includes a first roller, a first roller shaft coupled to the first roller, a first locking mechanism coupled to the first roller shaft, a second roller, a second roller shaft coupled to the second roller and a second locking mechanism coupled to the second roller shaft. The mobile support system further includes a body comprising a housing and a carriage positioned within the housing, a support arm coupled to the carriage and extending vertically upward from the housing, the support arm configured to support the arm of the medical device and a mounting block coupled to the carriage proximate to the support arm, the mounting block configured to couple with the base of the arm of the medical device, the mounting block comprising a set of pins configured to couple with the first roller shaft and the second roller shaft, wherein the first locking mechanism and the second locking mechanism are configured to lock the first roller shaft and the second roller shaft, respectively, when the set of pins of the mounting block are disengaged from the first roller shaft and the second roller shaft.

### BRIEF DESCRIPTION OF THE DRAWINGS

This application will become more fully understood from the following detailed description, taken in conjunction with the accompanying figures, wherein like reference numerals refer to like elements in which:
FIG. 1 is a schematic diagram of an exemplary medical bed or table with a medical device mounted to the medical bed or table in accordance with an embodiment;
FIG. 2 is a perspective view of a mobile support system in a first position in accordance with an embodiment;
FIG.3 is a perspective view of a mobile support system in a second position in accordance with an embodiment;
FIG. 4 is a perspective front view of a portion of a mobile support system in accordance with an embodiment;
FIG. 5 is a perspective view of a mobile support system in a first position in accordance with an embodiment;
FIG. 6 is a perspective view of a mobile support system with a raised carriage at the first position in accordance with an embodiment;
FIG. 7 is a perspective view of a mobile support system in a second position in accordance with an embodiment;
FIG. 8 is a perspective view of a mobile support system with a lowered carriage at the second position in accordance with an embodiment;
FIG. 9 is a perspective view looking upward towards a bottom portion of a base of an arm of a medical device in accordance with an embodiment;
FIG. 10 is a side view of a mounting block in a carriage of a mobile support system in accordance with an embodiment;
FIG. 11 is a front view of a mobile support system in accordance with an embodiment;
FIG. 12 is a perspective view of a mobile support system with a lowered carriage at the second position in accordance with an embodiment;
FIG. 13 is a perspective view of a mobile support system in a second position in accordance with an embodiment;
FIG. 14 is a perspective view of a mobile support system with a raised carriage at the first position in accordance with an embodiment; and
FIG. 15 is a perspective view of a mobile support system in a first position in accordance with an embodiment.

### DETAILED DESCRIPTION OF THE PREFERRED EMBODIMENTS

In the following description, the vertical direction is the direction parallel to the direction of gravity and the horizontal direction is a direction generally perpendicular to the direction of gravity. The term upward will be a vertical direction opposite the direction of the force of gravity and the term downward will be a vertical direction in the direction of gravity.

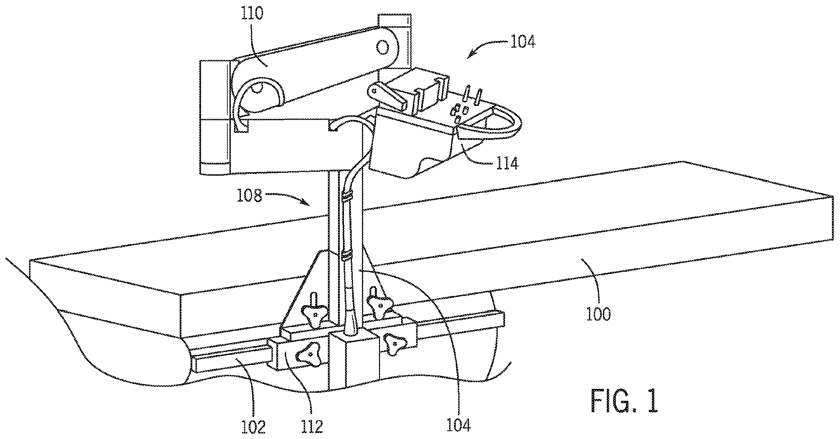

FIG. 1 is a schematic diagram of an exemplary medical bed or table with a medical device mounted to the medical bed or table in accordance with an embodiment. In FIG. 1, a medical bed or table 100 includes a rail 102. A medical device 104 may be mounted to the table 100 using the rail 102. The medical device 104 includes an arm 106 that has a lower portion 108 and is used to support an instrument or system used in a medical procedure. In an embodiment, the arm 106 may include an articulated upper portion 110. The arm 106 also includes a base 112 that is configured to mount and secure the medical device 104 to the rail 102. The instrument or system supported by the arm 106 may be, for example, a robotic catheter system, an IV pole, a monitor, a contrast solution injector, etc. In FIG. 1, an exemplary robotic catheter system 114 is shown attached to the arm 106.

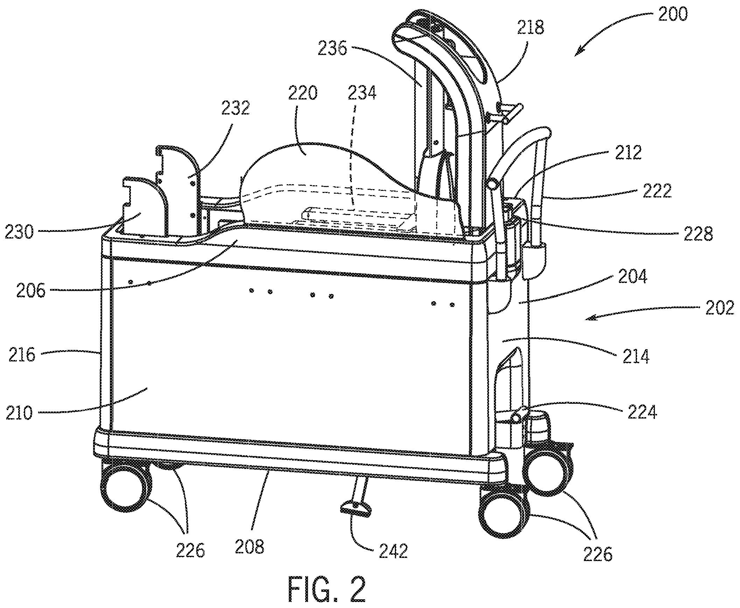

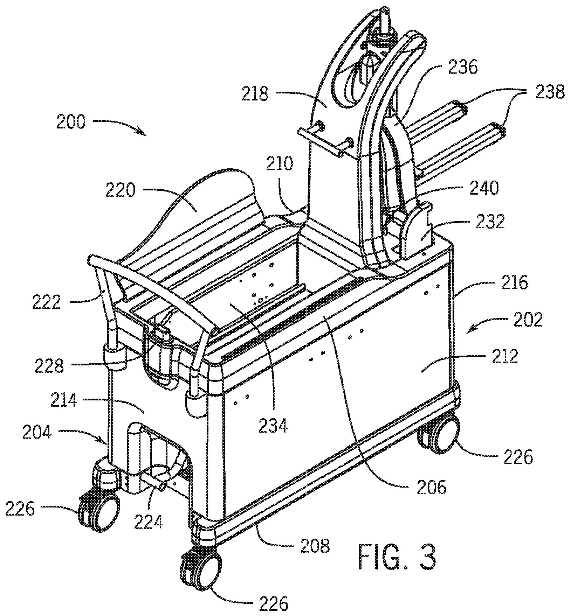

When the medical device 104 is not mounted to the table 100, the medical device 104 may be transported (to and from the table 100) and stored using a mobile support as described below with respect to Figs. 2-15. The mobile support may also be used to install and remove the medical device 104 from the table 100. FIG. 2 is a perspective view of a mobile support system in a first position in accordance with an embodiment and FIG.3 is a perspective view of a mobile support system in a second position in accordance with an embodiment. Referring to FIGs. 2 and 3, a mobile support 200 (e.g., a cart) includes a body 202 having a housing 204. The housing 204 includes a top surface 206, a bottom surface 208, a first side 210, a second side 212, a first end 214 and a second end 216. While a rectangular or cube shaped housing is shown, in other embodiments, the housing of the mobile support system 200 may have other shapes such as a cylinder and the housing may be a closed or open frame. A set of wheels 236 are attached to the bottom surface 208 of the housing 204. A floor brake 242 is also positioned on the bottom surface and is configured to be in a raised position when the mobile support 200 is used to transport and store the medical device and in a lowered position when the mobile support 200 is used to install the medical device on the rail of a bed or table and to remove the medical device from the rail of a bed or table as discussed further below.

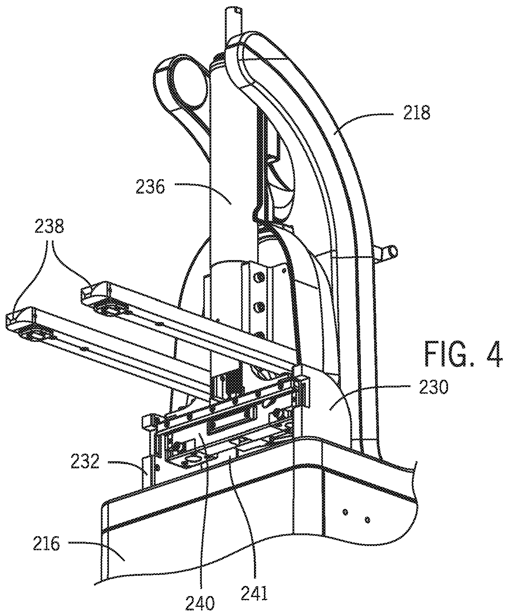

The first end 214 of the housing includes a handle 222, a foot pedal 224 and a button 228. A support arm 218 is positioned in a carriage 234 inside the housing 204. In FIG. 2, the support arm 218 is located in a first position proximate the first end 214 of the housing 204. The first position shown in FIG. 2 may be used when the medical device is not in use and is being stored. In FIG. 3, the support arm 218 is located at a second position proximate the second end 216 of the housing 204. The second position shown in FIG. 3, may be used when the mobile support 200 is being used to install the medical device on or remove the medical device from the bed or table. A lower portion 236 of an arm of a medical device (e.g., lower portion 108 of arm 106 shown in FIG. 1) may be positioned in and adjacent to the support arm 218. For clarity, the remainder of the medical device (e.g., articulated portion 110 and instrument 114 shown in FIG. 1) is not shown. The lower portion 236 of the arm is removably attached to the support arm 218 (and the mobile support 200). For example, the support arm 218 may include a mounting block 241 (shown in FIG. 4) at a lower end of the support arm 218 proximate the top surface 206 of the housing and the carriage 234. Referring to FIG. 4, the lower portion 236 of the arm of the medical device includes a base 240 that is configured to engage the mounting block 241 when the medical device is mounted on the cart. In embodiments described below, the base 240 and mounting block 241 interface vertically. The base 240 is also configured to engage the rail of a bed or table when the medical device is mounted to the bed or table. The lower portion 236 of the arm also includes a set of feet 238 that may be located on top of the bed or table and under, for example, a mattress when the medical device is mounted and secured to the bed or table. The feet 238 may be used to apply load directly onto the table surface in addition to a rail on the table.

In FIG. 2, a side guide 220 is shown positioned along the top surface between the first end 214 and the second end 216 proximate to the first side 210 of the housing. In another embodiment, a side guide 220 may also be positioned along the top surface between the first end 214 and the second end 216 proximate to the second side 212. The side guide(s) 220 constrain and limit the range of motion of the support arm 218 and the arm 236 as they are moved from the first end 214 to the second end 216 of the housing 204 within the carriage 234. Limiting the range of motion of the support arm 218 and the arm 236 may result in increased stability of the mobile support system 200. A first rail detect guide 230 and a second rail detect guide 232 are located along the top surface proximate to the second end 216 of the housing. The first 230 and second 232 rail detect guide are configured to determine if the mobile support 200 and the arm 236 are in the correct position in relation to the rail of the bed or table for installation or removal of the medical device as described further below.

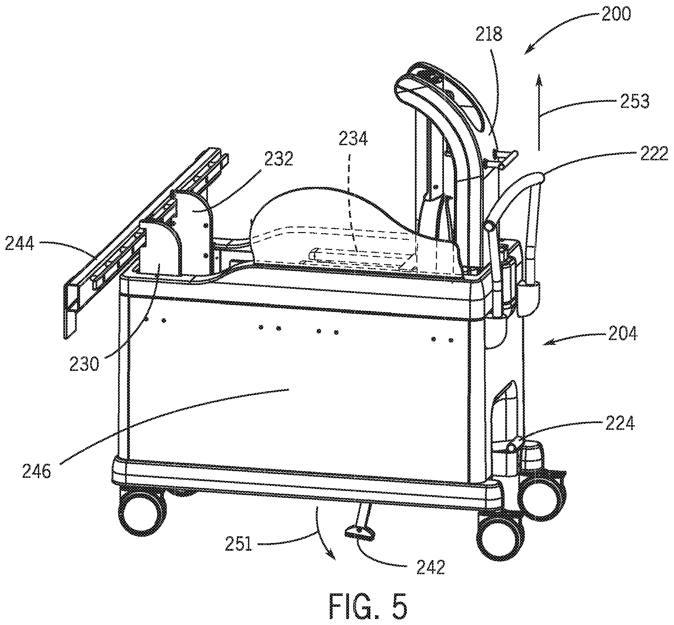

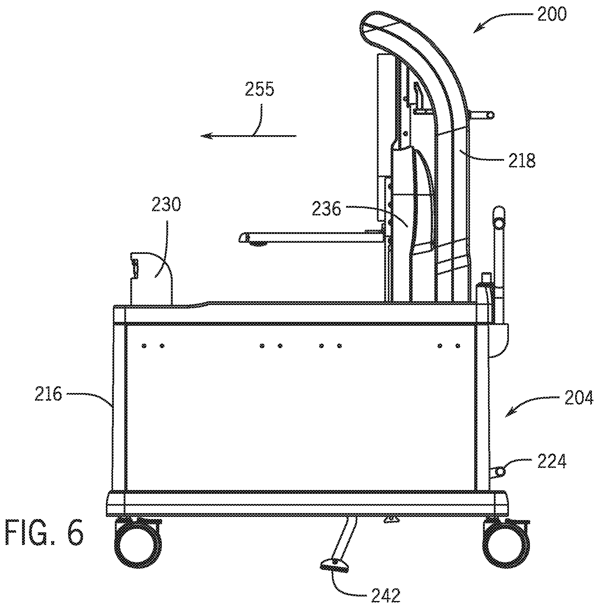

To install the medical device on the rail of a bed or table, the mobile support 200 may be positioned next to the side of the bed adjacent to the rail on the table. FIG. 5 is a perspective view of a mobile support system in a first position in accordance with an embodiment. In FIG. 5, the mobile support 200 is wheeled up to the table so that the first rail detect guide 230 and the second rail detect guide 232 contact a rail 244 on the bed or table. For clarity, only the rail 244 of the bed or table is shown. The first rail detect guide 230 and the second rail detect guide 232 ensure that the table and rail 244 are in a correct height range. In addition, the first 230 and second 232 rail detect guides each include a link or switch that when actuated by contact with the rail 244 activate the mechanisms (e.g., hydraulics) used to raise and lower the carriage 234 (and thereby the support arm 218 and lower portion 236 of the medical device arm) so that the medical device may be installed onto the rail 244. In one embodiment, actuations of the links or switches in the first 230 and second 232 rail detect guides by contact with the rail 244 causes a bypass valve in a hydraulic circuit to close and switch the foot pedal 224 from a bypass mode to an actuation mode. In the bypass mode, the foot pedal 224 and mechanisms used to raise and lower the carriage 234 in the housing 204 of the mobile support 200 are inactive. In the actuation mode, the foot pedal 224 and the mechanisms used to raise and lower the carriage 234 are activated and a user may, for example, pump the foot pedal 224 to raise the carriage 234, support arm 218 and arm 236 upward in a vertical direction as indicated by arrow 253. A mechanism 246 may be used to raise the carriage 234 in response to the actuation of the foot pedal 224, for example, a scissor mechanism or lift as shown in FIG. 5. In various embodiments, the foot pedal 224 may be, for example, hydraulic or a mechanical linkage. In addition, actuation of the foot pedal 224 may cause the floor brake 242 to lower to contact the floor (as shown by arrow 251) and force or urge the mobile support 200 against the rail 244. FIG. 6 shows the carriage, support arm 218 and arm 236 in a raised position at the first position at the first end 214 of housing 204.

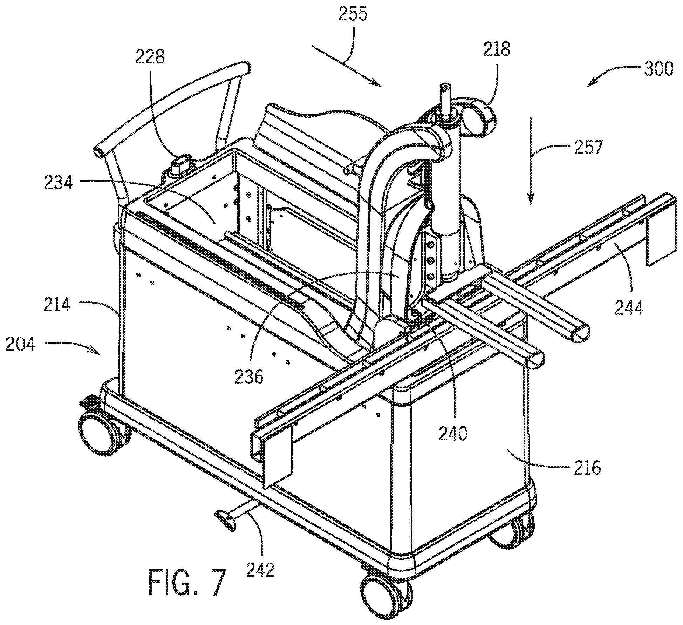

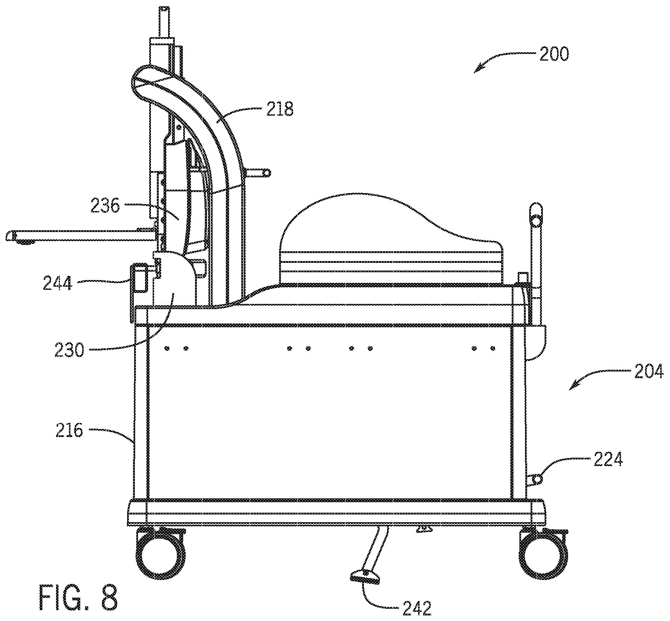

A user may then slide the support arm 218, arm 236 and carriage 234 from the first position proximate the first end 214 of the housing 204 to a second position as shown by arrow 255 in FIG. 6. FIG. 7 is a perspective view of a mobile support system in a second position in accordance with an embodiment. In FIG. 7, the support arm 218, arm 236 and carriage 234 have been moved in a horizontal direction (as shown by arrow 255) to the second position proximate the second end 216 of the housing 204. In one embodiment, the horizontal movement may be provided using linear bearings (not shown). A bias spring (not shown) may be used to push or force the carriage 234 towards the second position and hold the carriage 234 in the second position. In an embodiment, the bias spring may be implemented as an over center spring. In another embodiment, the bias spring may be implemented as a spring-loaded cam follower on a linear cam. The carriage 234, support arm 218 and arm 236 may then be lowered, for example, by actuating a button 228, towards the rail 244 (as shown by arrow 257) so that the arm 236 and base 240 are positioned on the rail 244. As mentioned above, a mechanism such as a scissor mechanism may be used to lower the carriage 234. When the base 240 is positioned and secured on the rail 244, the carriage 234 continues to be lowered downwards to disengage from the base 240 of the arm 236. FIG. 8 shows the carriage and support arm 218 is a lowered position in second position at the second end 216 of the housing 204. The mobile support 200 may then be wheeled away from the bed or table.

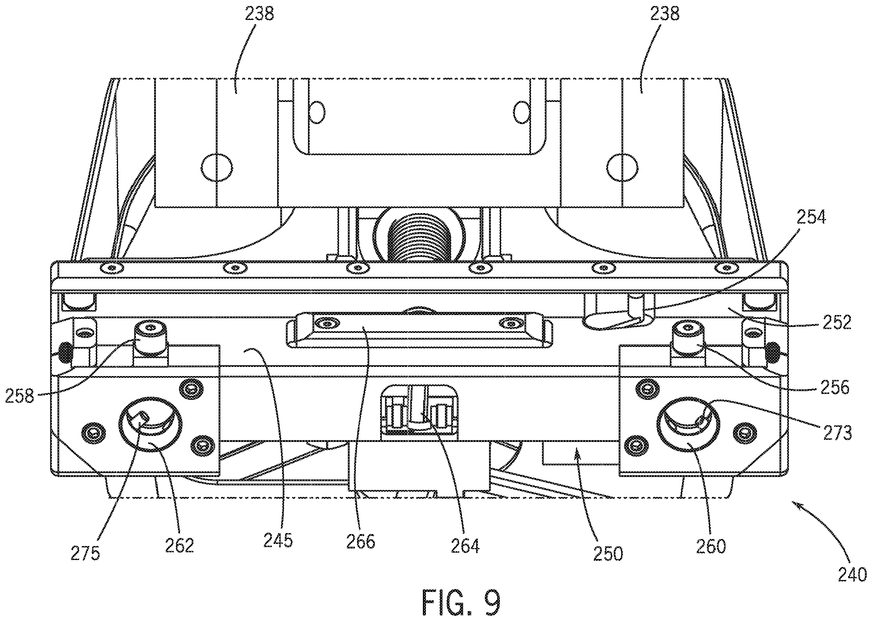

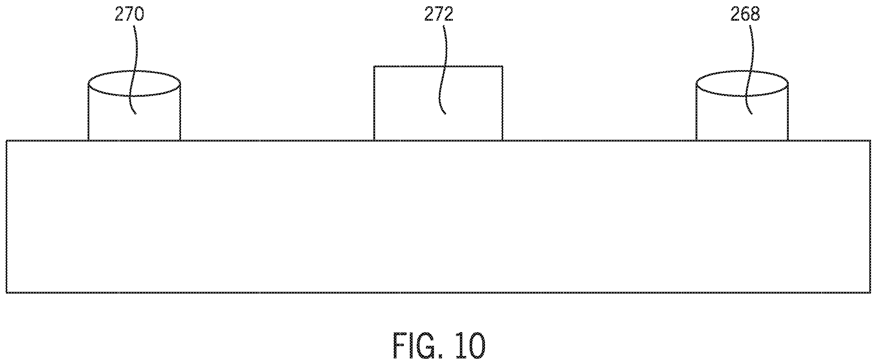

It is desirable to keep the medical device locked onto the mobile support 200 until the arm 236 is properly seated on the rail 244 for installation on the rail 244 and that the medical device remains secured to the rail 244 until the mobile support 200 is properly positioned below the arm 236 for removal from the rail 244. As mentioned above, the base 240 of the arm 236 engages with the mounting block 241 on the mobile support 200. FIG. 9 is a perspective view looking upward towards a bottom portion of a base of an arm of a medical device in accordance with an embodiment and FIG. 10 is a side view of a mounting block in a carriage of a mobile support system in accordance with an embodiment. Referring to FIG. 9, base 240 includes a set of feet 238, a lip 252, a front side 245 and a bottom surface 250. A rail detect lever 254 is located on a bottom surface of lip 252. The rail detect lever 254 is used to make sure the arm base 240 is fully seated on the rail 244 (not shown) before the arm base 240 detaches from the mobile support 200. When the arm base 240 is lowered onto the rail, the rail detect lever 254 makes contact with the rail and either unlocks a first roller shaft 260 or a second roller shaft 262. In another embodiment, both the first roller shaft 260 and the second roller shaft 262 are locked and are unlocked when the rail detect lever 254 makes contact with the rail. When the base 240 and the mounting block 241 are engaged, a first pin 268 and a second pin 270 are positioned in the first roller shaft 260 and the second roller shaft 262, respectively. First pin 268 and second pin 270 are shown in FIG. 10 having a cylindrical or round shape, however, other shapes may be used for the pins 268 and 270. The first 268 and second 370 pins of the mounting block 241 are shown in FIG. 10. Referring again to Fig. 9, base 240 also includes a first roller 256 and a second roller 258 that are coupled to the first roller shaft 260 and the second roller shaft 262, respectively. When the base 240 and mounting block 241 are engaged, the first roller 256 and the second roller 258 are in a position (not shown in Fig. 9) so they lie substantially parallel to the first side 245 of the base 240 and do not protrude outward from and perpendicular to the first side 245 of the base 240. Once the first 260 and second 262 roller shafts are unlocked, the mobile support 200 can continue to be lowered so as to disengage the first 268 and second 270 pin of the mounting block 241 from base 240. As the pin 268 is removed, a follower pin 273 in first roller shaft 260 rotates the roller shaft 260 to move the first roller 256 to a position (shown in FIG. 9) where it protrudes outward from and perpendicular to the first side 245 of base 240 so that the first roller 256 lies under the rail (not shown). As the pin 270 is removed, a follower pin 275 in second roller shaft 262 rotates the roller shaft 262 to move the second roller 258 to a position (shown in FIG. 9) where it protrudes outward from and perpendicular to the first side 245 of base 240 so that the second roller 258 lies under the rail (not shown). First roller 256 and second roller 258 secure or lock the base 240 onto the rail. When pins 268 and 270 disengage from the roller shafts 260 and 262 respectively, the follower pins 273 and 275 slide inwards and engage a locking mechanism that prevents roller shafts 260 and 262 from rotating.

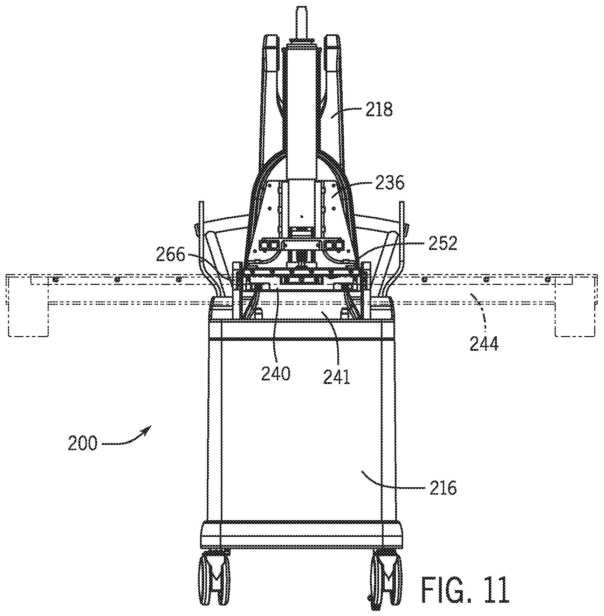

Base 240 also includes a friction brake 266 positioned on the first side 245 of the base 240 and a brake release 264 positioned in the bottom surface 250 of the base 240. When the base 240 and mounting block 241 (shown in Fig. 10) are engaged, a brake release pin 272 (shown in FIG. 10) on the mounting block 241 is positioned in the brake release 264. Brake release pin 272 is shown in FIG. 10 having a rectangular shape, however, other shapes may be used for the brake release pin 272. As the brake release pin 272 is removed from the brake release as the mobile support 200 is lowered, the brake 266 is applied and forced against the rail (e.g., a spring-loaded brake) to secure the base 240 to the rail as shown in FIG. 11. FIG. 11 is a front view of a mobile support system in accordance with an embodiment. The support arm 218 and arm 236 are in the second position proximate to the second end 216 of the mobile support 200. The base 240 of the arm 236 is seated on the rail 244 and the brake 266 is against the rail 244. As the carriage 234 is lowered, the floor brake 242 is released and raises up from the floor. Once the carriage 234 of the mobile support 200 is lowered to disengage the mounting block 241 from the base 240, the mobile support 200 may then be wheeled away from the bed or table. The medical device mounted to the table is shown in FIG. 1.

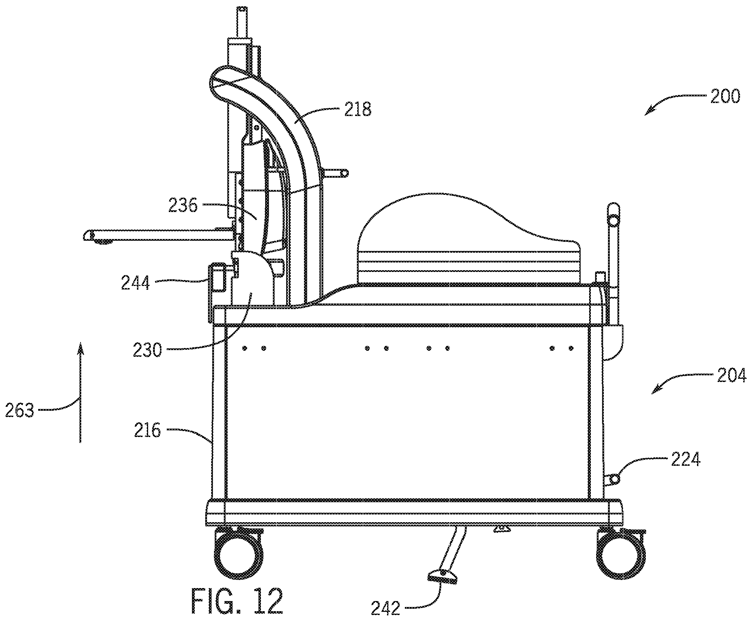

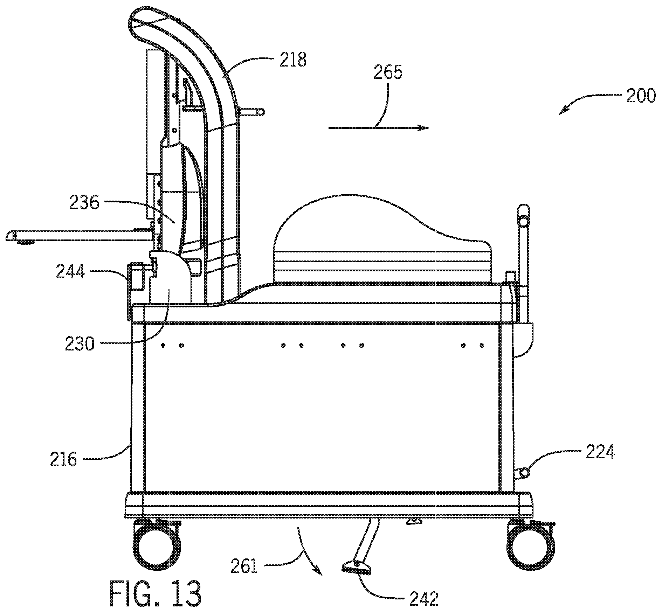

To remove the medical device from the rail of a bed or table, the mobile support 200 may be positioned next to the side of the bed adjacent to the rail on the table. FIG. 12 is a perspective view of a mobile support system with a lowered carriage in the second position and FIG. 13 is a perspective view of a mobile support system with a raised carriage in the second position . Referring to FIGs. 12 and 13, the mobile support 200 is wheeled up to the table so that the first rail detect guide 230 and the second rail detect guide 232 contact the rail 244 on the bed or table. For clarity, only the rail 244 of the bed or table is shown. As discussed above with respect to FIG. 5, the first 230 and second 232 rail detect guides each include a link or switch that when actuated by contact with the rail 244 unlock the mechanisms (e.g., hydraulics) used to raise and lower the carriage 234 (and thereby the support arm 218 and lower portion 236 of the medical device arm) so that the medical device may be removed from the rail 244. Once the foot pedal 224 and the mechanisms used to raise and lower the carriage 234 are unlocked, a user may, for example, pump the foot pedal 224 to raise the carriage 234, support arm 218 and arm 236 upward in a vertical direction as indicated by arrow 263. In addition, actuation of the foot pedal 224 causes the floor brake to lower to contact the floor (as shown by arrow 261) and force or urge the mobile support 200 against the rail 244. As the carriage 234 is raised, the mounting block 241 on the mobile support 200 engages the base 240 of the arm 236.

Referring again to FIGs. 9 and 10, when the base 240 and the mounting block 241 are engaged, a first pin 268 and a second pin 270 are positioned in the first roller shaft 260 and the second roller shaft 262, respectively. As the first pin 268 is inserted into the first roller shaft 260, the follower pin 273 in first roller shaft 260 first unlocks the roller shaft 260 and then rotates the roller shaft 260 to move the first roller 256 to a position (not shown in Fig. 9) so it lies substantially parallel to the first side 245 of the base 240 and does not protrude outward from and perpendicular to the first side 245 of the base 240. As the second pin 270 is inserted into the second roller shaft 262, the follower pin 275 in second roller shaft 262 first unlocks the roller shaft 262 and then rotates the roller shaft 262 to move the second roller 258 to a position (not shown in Fig. 9) so it lies substantially parallel to the first side 245 of the base 240 and does not protrude outward from and perpendicular to the first side 245 of the base 240. In addition, as the base 240 and mounting block 241 are engaged, the brake release pin 272 (shown in FIG. 10) on the mounting block 241 is positioned in the brake release 264. As the brake release pin 272 is inserted in the brake release 264 as the mobile support 200 is raised, the brake 266 is released and no longer applies a force to the rail 244. In an embodiment, the brake release pin 272 makes contact with a rocker in brake release 264 and the rocker may be attached to the brake with a tie rod. Once the base 240 and the mounting block 241 are engaged, the roller shafts 260, 262 are unlocked, rollers 256, 258 are rotated out of the position underneath the rail and the brake is released. As the arm base 240 is raised off of the rail the rail detect lever 254 disengages from the rail (e.g., no longer makes contact with the rail) and at least one of the first roller shaft 260 and the second roller shaft 262 are locked.

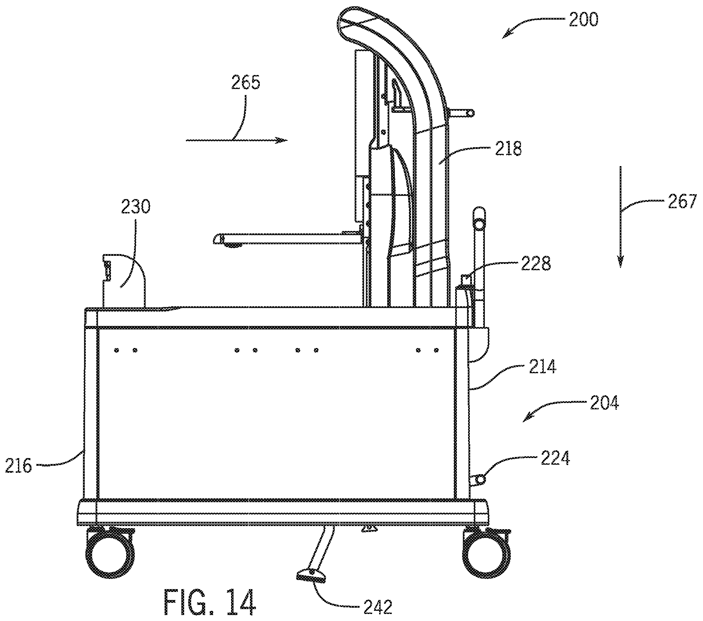

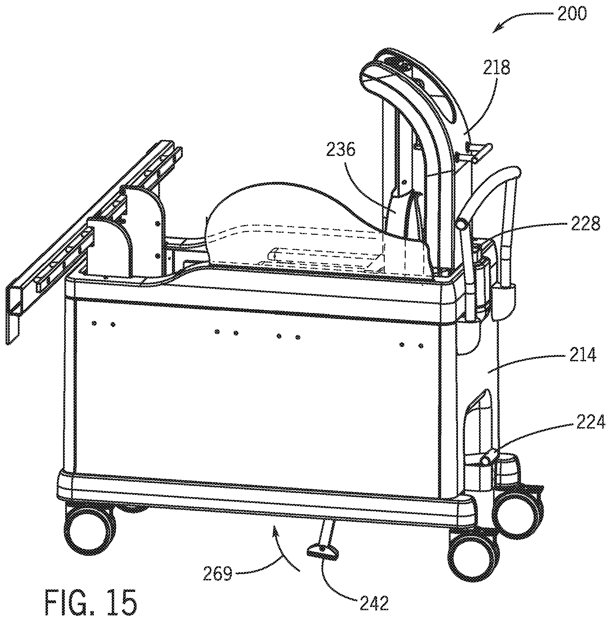

Returning to FIG. 13, the carriage 234 of mobile support 200 may then continue to be raised until the arm 236 and support arm 218 are a sufficient distance above the table. A user may then slide (as shown by arrow 265) the support arm 218, arm 236 and carriage 234 from the second position proximate the second end 216 of the housing 204 to a first position as shown in FIG. 14. FIG. 14 is a perspective view of a mobile support system with a raised carriage at the first position in accordance with an embodiment . In FIG. 14, the support arm 218, arm 236 and carriage 234 have been moved in a horizontal direction (as shown by arrow 265) to the first position proximate the first end 214 of the housing 204. In one embodiment, the horizontal movement may be provided using linear bearings (not shown). The carriage 234, support arm 218 and arm 236 may then be lowered (as shown by arrow 267), for example, by actuating a button 228, into the housing so that the arm 236 and base 240 are in a storage position as shown in Fig. 15. As mentioned above, a mechanism such as a scissor mechanism may be used to lower the carriage 234. Referring to Fig. 15, when the support arm 218 and arm 236 are in the storage position, the floor brake 242 releases and moves to a raised position (as shown by arrow 269). The mobile support 200 may then be wheeled away from the bed or table.

This written description used examples to disclose the invention, including the best mode, and also to enable any person skilled in the art to make and use the invention. The patentable scope of the invention is defined by the claims, and may include other examples that occur to those skilled in the art. The order and sequence of any process or method steps may be varied or re-sequenced according to alternative embodiments.

The scope of the invention is defined by the appended claims.

## Claims

1. A mobile support system (200) comprising:
a medical device (104) having an arm (236) with a base (240), the base (240) comprising:
a first roller (256);
a first roller shaft (260) coupled to the first roller (256);
a first locking mechanism coupled to the first roller shaft (260);
a second roller (258);
a second roller shaft (262) coupled to the second roller (258); and
a second locking mechanism coupled to the second roller shaft (262);
a body (202) comprising a housing (204) and a carriage (234) positioned within the housing (204);
a support arm (218) coupled to the carriage (234) and extending vertically upward from the housing (204), the support arm configured to support the arm of the medical device (104); and
a mounting block (241) coupled to the carriage (234) proximate to the support arm, the mounting block (241) configured to couple with the base (240) of the arm of the medical device (104), the mounting block (241) comprising a set of pins (268, 270) configured to couple with the first roller shaft (260) and the second roller shaft (262), wherein the first locking mechanism and the second locking mechanism are configured to lock the first roller shaft (260) and the second roller shaft (262), respectively, when the set of pins (268,270) of the mounting block (241) are disengaged from the first roller shaft (260) and the second roller shaft (262) and wherein the first locking mechanism and the second locking mechanism are configured to unlock the first roller shaft (260) and the second roller shaft (262), respectively, when the set of pins ( 268, 270) of the mounting block (241) are engaged with the first roller shaft (260) and the second roller shaft (262).

2. The mobile support system (200) according to claim 1, wherein the first locking mechanism comprises a follower pin (273) disposed within the first roller shaft (260).

3. The mobile support system (200) according to 1, wherein the second locking mechanism comprises a follower pin (275) disposed within the second roller shaft (262).

4. The mobile support system (200) according to claim 1, wherein the first roller (256) and the second roller (258) are configured to move from a first position to a second position when the set of pins (268, 270) of the mounting block (241) are engaged with the first roller shaft (260) and the second roller shaft (262).

5. The mobile support system (200) according to claim 1, further comprising:
wherein the base (240) comprises a rail detect lever (254) configured to be actuated when contact is made between the rail detect lever (254) and a surface.

6. The mobile support system (200) according to claim 5, wherein the surface is a surface of a rail (244) of a table.

7. The mobile support system (200) according to claim 5 or 6, wherein the first roller shaft (260) and the second roller shaft (262) are configured to be unlocked when the rail detect lever (254) is actuated.

8. The mobile support system (200) according to any one of claims 5 - 7, wherein the first locking mechanism comprises a follower pin (273) disposed within the first roller shaft (260)

9. The mobile support system (200) according to any one of claims 5 - 8, wherein the second locking mechanism comprises a follower pin (275) disposed within the second roller shaft (262).

10. The mobile support system (200) according to claim 9, wherein the first roller (256) and the second roller (258) are configured to move from a first position to a second position when the set of pins (268, 270) of the mounting block (241) are engaged with the first roller shaft (260) and the second roller shaft (262).

11. The mobile support system (200) according to any one of claims 1 - 10, wherein when the base (240) of the arm (236) is lowered onto the rail (244), the rail detect lever (254) makes contact with the rail (244) and either unlocks the first roller shaft (260) or the second roller shaft (262).

12. The mobile support system (200) according to any one of claims 1 - 10, wherein when the base (240) of the arm (236) is lowered onto the rail (244), the rail detect lever (254) makes contact with the rail (244) and locks or unlocks both the first roller shaft (260) and the second roller shaft (262).

13. The mobile support system (200) according to any one of claims 1 - 12, wherein the medical device (104) includes a robotic catheter system, an IV pole, a monitor, or a contrast solution injector.

## Patentansprüche

1. Mobiles Trägersystem (200) umfassend:
eine medizinische Vorrichtung (104) mit einem Arm (236) mit einer Basis (240), wobei die Basis (240) umfasst:
eine erste Rolle (256);
eine erste Rollenwelle (260), die an die erste Rolle (256) gekoppelt ist;
einen ersten Verriegelungsmechanismus, der an die erste Rollenwelle (260) gekoppelt ist;
eine zweite Rolle (258);
eine zweite Rollenwelle (262), die an die zweite Rolle (258) gekoppelt ist; und
einen zweiten Verriegelungsmechanismus, der an die zweite Rollenwelle (262) gekoppelt ist;
einen Körper (202), umfassend ein Gehäuse (204) und einen in dem Gehäuse (204) angeordneten Schlitten (234);
einen Trägerarm (218), der an den Schlitten (234) gekoppelt ist und sich vom Gehäuse (204) vertikal nach oben erstreckt, wobei der Trägerarm zum Tragen des Arms der medizinischen Vorrichtung (104) ausgelegt ist; und
einen Montageblock (241), der an den Schlitten (234) in der Nähe des Trägerarms gekoppelt ist, wobei der Montageblock (241) dazu ausgelegt ist, mit der Basis (240) des Arms der medizinischen Vorrichtung (104) gekoppelt zu werden, wobei der Montageblock (241) einen Satz von Stiften (268, 270) umfasst, die dazu ausgelegt sind, mit der ersten Rollenwelle (260) und der zweiten Rollenwelle (262) gekoppelt zu werden, wobei der erste Verriegelungsmechanismus und der zweite Verriegelungsmechanismus dazu ausgelegt sind, die erste Rollenwelle (260) bzw. die zweite Rollenwelle (262) zu verriegeln, wenn der Satz von Stiften (268, 270) des Montageblocks (241) von der ersten Rollenwelle (260) und der zweiten Rollenwelle (262) gelöst ist, und wobei der erste Verriegelungsmechanismus und der zweite Verriegelungsmechanismus dazu ausgelegt sind, die erste Rollenwelle (260) bzw. die zweite Rollenwelle (262) zu entriegeln, wenn der Satz von Stiften (268, 270) des Montageblocks (241) mit der ersten Rollenwelle (260) und der zweiten Rollenwelle (262) in Eingriff steht.

2. Mobiles Trägersystem (200) nach Anspruch 1, wobei der erste Verriegelungsmechanismus einen Mitnehmerstift (273) umfasst, der in der ersten Rollenwelle (260) angeordnet ist.

3. Mobiles Trägersystem (200) nach Anspruch 1, wobei der zweite Verriegelungsmechanismus einen Mitnehmerstift (275) umfasst, der in der zweiten Rollenwelle (262) angeordnet ist.

4. Mobiles Trägersystem (200) nach Anspruch 1, wobei die erste Rolle (256) und die zweite Rolle (258) dazu ausgelegt sind, sich aus einer ersten Position in eine zweite Position zu bewegen, wenn der Satz von Stiften (268, 270) des Montageblocks (241) mit der ersten Rollenwelle (260) und der zweiten Rollenwelle (262) in Eingriff steht.

5. Mobiles Trägersystem (200) nach Anspruch 1, ferner umfassend:
wobei die Basis (240) einen Schienenerkennungshebel (254) umfasst, dazu ausgelegt, betätigt zu werden, wenn Kontakt zwischen dem Schienenerkennungshebel (254) und einer Oberfläche erfolgt.

6. Mobiles Trägersystem (200) nach Anspruch 5, wobei die Oberfläche eine Oberfläche einer Schiene (244) eines Tisches ist.

7. Mobiles Trägersystem (200) nach Anspruch 5 oder 6, wobei die erste Rollenwelle (260) und die zweite Rollenwelle (262) dazu ausgelegt sind, entriegelt zu werden, wenn der Schienenerkennungshebel (254) betätigt wird.

8. Mobiles Trägersystem (200) nach einem der Ansprüche 5-7, wobei der erste Verriegelungsmechanismus einen Mitnehmerstift (273) umfasst, der in der ersten Rollenwelle (260) angeordnet ist.

9. Mobiles Trägersystem (200) nach einem der Ansprüche 5-8, wobei der zweite Verriegelungsmechanismus einen Mitnehmerstift (275) umfasst, der in der zweiten Rollenwelle (262) angeordnet ist.

10. Mobiles Trägersystem (200) nach Anspruch 9, wobei die erste Rolle (256) und die zweite Rolle (258) dazu ausgelegt sind, sich aus einer ersten Position in eine zweite Position zu bewegen, wenn der Satz von Stiften (268, 270) des Montageblocks (241) mit der ersten Rollenwelle (260) und der zweiten Rollenwelle (262) in Eingriff steht.

11. Mobiles Trägersystem (200) nach einem der Ansprüche 1-10, wobei, wenn die Basis (240) des Arms (236) auf die Schiene (244) abgesenkt wird, der Schienenerkennungshebel (254) mit der Schiene (244) in Kontakt kommt und entweder die erste Rollenwelle (260) oder die zweite Rollenwelle (262) entriegelt.

12. Mobiles Trägersystem (200) nach einem der Ansprüche 1-10, wobei, wenn die Basis (240) des Arms (236) auf die Schiene (244) abgesenkt wird, der Schienenerkennungshebel (254) mit der Schiene (244) in Kontakt kommt und sowohl die erste Rollenwelle (260) als auch die zweite Rollenwelle (262) verriegelt oder entriegelt.

13. Mobiles Trägersystem (200) nach einem der Ansprüche 1-12, wobei die medizinische Vorrichtung (104) ein Roboterkathetersystem, eine IV-Stange, einen Monitor oder einen Kontrastlösungsinjektor umfasst.

## Revendications

1. Système de support mobile (200) comprenant :
un dispositif médical (104) comportant un bras (236) avec une base (240), la base (240) comprenant :
un premier rouleau (256) ;
un premier arbre de rouleau (260) couplé au premier rouleau (256) ;
un premier mécanisme de verrouillage couplé au premier arbre de rouleau (260) ;
un second rouleau (258) ;
un second arbre de rouleau (262) couplé au second rouleau (258) ; et
un second mécanisme de verrouillage couplé au second arbre de rouleau (262) ;
un corps (202) comprenant un boîtier (204) et un chariot (234) positionné à l'intérieur du boîtier (204) ;
un bras de support (218) couplé au chariot (234) et s'étendant verticalement vers le haut à partir du boîtier (204), le bras de support étant configuré pour supporter le bras du dispositif médical (104) ; et
un bloc de montage (241) couplé au chariot (234) à proximité du bras de support, le bloc de montage (241) étant configuré pour s'accoupler avec la base (240) du bras du dispositif médical (104), le bloc de montage (241) comprenant un ensemble de goupilles (268, 270) configurées pour s'accoupler avec le premier arbre de rouleau (260) et le second arbre de rouleau (262), le premier mécanisme de verrouillage et le second mécanisme de verrouillage étant configurés pour verrouiller respectivement le premier arbre de rouleau (260) et le second arbre de rouleau (262), lorsque l'ensemble de goupilles (268, 270) du bloc de montage (241) est désengagé du premier arbre de rouleau (260) et du second arbre de rouleau (262), et le premier mécanisme de verrouillage et le second mécanisme de verrouillage étant configurés pour déverrouiller respectivement le premier arbre de rouleau (260) et le second arbre de rouleau (262), lorsque l'ensemble de goupilles (268, 270) du bloc de montage (241) sont en prise avec le premier arbre de rouleau (260) et le second arbre de rouleau (262).

2. Système de support mobile (200) selon la revendication 1, le premier mécanisme de verrouillage comprenant une goupille de suivi (273) disposée à l'intérieur du premier arbre de rouleau (260).

3. Système de support mobile (200) selon la revendication 1, le second mécanisme de verrouillage comprenant une goupille de suivi (275) disposée à l'intérieur du second arbre de rouleau (262).

4. Système de support mobile (200) selon la revendication 1, le premier rouleau (256) et le second rouleau (258) étant configurés pour se déplacer d'une première position à une seconde position lorsque l'ensemble de goupilles (268, 270) du bloc de montage (241) sont en prise avec le premier arbre de rouleau (260) et le second arbre de rouleau (262).

5. Système de support mobile (200) selon la revendication 1, comprenant en outre :
la base (240) comprenant un levier de détection de rail (254) configuré pour être actionné lorsqu'un contact est établi entre le levier de détection de rail (254) et une surface.

6. Système de support mobile (200) selon la revendication 5, la surface étant une surface d'un rail (244) d'une table.

7. Système de support mobile (200) selon la revendication 5 ou 6, le premier arbre de rouleau (260) et le second arbre de rouleau (262) étant configurés pour être déverrouillés lorsque le levier de détection de rail (254) est actionné.

8. Système de support mobile (200) selon l'une quelconque des revendications 5 à 7, le premier mécanisme de verrouillage comprenant une goupille de suivi (273) disposée à l'intérieur du premier arbre de rouleau (260).

9. Système de support mobile (200) selon l'une quelconque des revendications 5 à 8, le second mécanisme de verrouillage comprenant une goupille de suivi (275) disposée à l'intérieur du second arbre de rouleau (262).

10. Système de support mobile (200) selon la revendication 9, le premier rouleau (256) et le second rouleau (258) étant configurés pour se déplacer d'une première position à une seconde position lorsque l'ensemble de goupilles (268, 270) du bloc de montage (241) sont en prise avec le premier arbre de rouleau (260) et le second arbre de rouleau (262).

11. Système de support mobile (200) selon l'une quelconque des revendications 1 à 10, lorsque la base (240) du bras (236) est abaissée sur le rail (244), le levier de détection de rail (254) entrant en contact avec le rail (244) et déverrouillant soit le premier arbre de rouleau (260), soit le second arbre de rouleau (262).

12. Système de support mobile (200) selon l'une quelconque des revendications 1 à 10, lorsque la base (240) du bras (236) est abaissée sur le rail (244), le levier de détection de rail (254) entrant en contact avec le rail (244) et verrouillant ou déverrouillant à la fois le premier arbre de rouleau (260) et le second arbre de rouleau (262).

13. Système de support mobile (200) selon l'une quelconque des revendications 1 à 12, le dispositif médical (104) comprenant un système de cathéter robotique, un pôle IV, un dispositif de surveillance ou un injecteur de solution de contraste.
